# EUROPEAN PATENT APPLICATION

(11) **EP 1 374 794 A1**
(43) Date of publication of application: **02.01.2004**
(21) Application number: 03380080.6
(22) Date of filing: 31.03.2003
(51) Int. Cl.: A61B 19/02, A61G 13/00

(54) **Medical supply support**

(30) Priority: 19.06.2002 ES 200201567 U
(71) Applicant: ORNALUX S.A., 33211 Gijon (Asturias) (ES)
(72) Inventor: Olabarri Bustillo, Francisco Javier, 33211 Gijon (Asturias) (ES)
(74) Representative: Davila Baz, Angel

(57) **Abstract**

A medical supply support of the type intended for housing different types of instruments and to supply intakes of different fluids as well as for electric power. The support is constituted of a head (1) provided with means for fixing the head to the ceiling of the room where it is incorporated, a head to which a first horizontal arm (3) is hinged, and to this arm, another or more also horizontal and hinged arms (3), the last of which is finished in a vertical and telescopic connection (5) connecting said arms to a vertically elongated container (6) susceptible to being adapted in both this direction and in the transversal direction and with any orientation. Both the head and the arms, the hinge joints (4) between them and the vertical and telescopic connection (5) are hollow, permitting passage through them of the channels corresponding to the different connections for fluids, electricity or others from the ceiling of the room.

## Description

### OBJECT OF THE INVENTION

The present invention refers to a support which has been especially designed for holding the conventionally necessary supplies in a medical center, structured as an extendible and adjustable element which can be fixed to the ceiling, which permits being adjusted for any practical need and which is capacitated not only as a support for the most varied objects of medical use, but rather also as a hidden channeling means for different types of fluid, electric, etc. conduits.

### BACKGROUND OF THE INVENTION

In certain medical services, it is necessary to have a wide range of supplies, ranging from different types of instruments to intakes for water, air, electricity, etc.

For those supplies of the first type, small rolling cabinets are used, whereas the intakes for those supplies of the second type are normally dispersed throughout the office or room.

There are supports incorporating all the aforementioned supplies and which are normally carried out on a pedestal suitably fixed to the floor, from which trays, chests of drawers, etc., emerge, which on many occasions even have an adjustable setting.

However, this type of supports pose a drawback basically focused on two aspects, on one hand, they constitute a significant obstruction on the floor, making certain tasks difficult, including proper hygiene of the floor, it being difficult to take the electric or fluid conduits to pedestal, since a significant construction job on the floor becomes necessary; and on the other hand, when they are hinged supports, the stability of the mobile elements thereof is precarious, shifting unduly, for example due to a small corporal impact. An added drawback is the difficulty in cleaning and thoroughly disinfecting the support itself due to the great profusion of concave corners and grooves defined by its mobile structure.

### DESCRIPTION OF THE INVENTION

The medical supply support proposed by the invention fully satisfactorily solves the aforementioned drawback in each and every one of the different aspects mentioned.

To do so, more specifically and according to one of the features of the invention, said support is associated to a head permitting the fixing and holding thereof to a ceiling, which means that the support completely leaves the floor of the room clear and which permits said support, in an inoperative condition, to significantly retract upwards, towards a position in which it not only frees up the floor, but rather it is possible to walk thereunder.

A series of hinged arms are fixed to said head for support with transversal movement possibility, in any direction, of a container, which is joined to the last of said arms through a vertical telescopic connection, permitting a significant shift in height of said container.

Both the fixing head and the hinged arms, their hinge joints and the vertical connection are hollow, which permits, through a suspended ceiling, that any type of conduits extending down to the lower container are hidden, a lower container where the corresponding intakes will emerge to the outside on any of the lateral sides of the container and in any desired location, with the collaboration of a series of removable covers normally blocking respective holes.

There are also fixing elements arranged on the inside of said holes which can be selectively used for the incorporation of removable trays, chests of drawers, or any other type of accessory deemed suitable, such that these elements can also be regulated in height.

In correspondence with the hinged connection between arms and vertical telescopic connection, respective inner bushings are arranged provided with a perimetral outer groove where a toric and inflatable chamber is housed, such that when in an empty state, said chamber permits the free swinging for said arms, whereas when pressure is supplied thereto, it acts as a brake element immobilizing them and, accordingly, duly stabilizing the entire support or any part thereof, as there is a single control for inflating all the inflatable chambers, or independent controls for each one of said chambers, which will be located in easily accessible areas, such as, for example, at the front of some of the removable trays or of the chest of drawers.

Handhole covers are arranged in the hinge joints of the arms which permit accessing the inside thereof in maintenance tasks and which, when closed, define a perfect superficial continuity for said joints which, together with the flat and rounded surface of the arms, with a cap finishing the head on the ceiling and with bushings associated to the lower final joint and to the upper base of the container, framing the ends of the vertical and telescopic connection, ensure for the assembly a perfectly smooth and continuous outer surface, easily cleanable and with no areas inclined to the depositing of dirt.

The disclosed structure is complemented or can be complemented with extensible elements which can be laterally fixed to the container, preferably in correspondence with its vertical edges, to place accessories such as infusion pumps, drainage tubes and other elements for the normal functioning of a medical center.

The container can be sectioned inside for the purpose of being able to make the intakes for gases, electricity or other fluids independent, such that said intakes are located on different sides of the container, for example, the network voltages being independent from the low voltages used in computers, audio and video.

### DESCRIPTION OF THE DRAWINGS

To complement the description being made and for the purpose of helping to better understand the features of the invention, according to a preferred practical embodiment example thereof, a set of drawings accompanies said description as an integral part thereof, which, with an illustrative and non-limiting character, shows the following:
Figure 1.- shows a perspective view of a medical supply support carried out according to the present invention.
Figure 2.- shows a cross-sectional or diametral view of a detail at the level of one of the hinged connection joints between arms.
Figure 3.- lastly shows another cross-sectional view of a detail, now at the level of the vertical telescopic connection between arms and container.

### PREFERRED EMBODIMENT OF THE INVENTION

In view of the indicated figures, it can be seen how the support proposed by the invention is structured as from a head (1) intended to be fixed to the ceiling of the room where said support is incorporated, a fixing which is hidden and finished with the collaboration of a cap (2), said head being hollow and hinged to one of the ends of a horizontal arm (3), which, on its other end and forming a joint (4), is hinged to a second horizontal arm (3'), which either directly or with the interposition of another arm, is also hinged to a vertical and telescopic connection (5), connecting the set of aforementioned arms (3) to a vertically elongated container (6) which, in the practical embodiment of the figures, adopts an equilateral, triangular-prismatic shape and with rounded vertices, but which can adopt any other shape without it affecting the essence of the invention whatsoever.

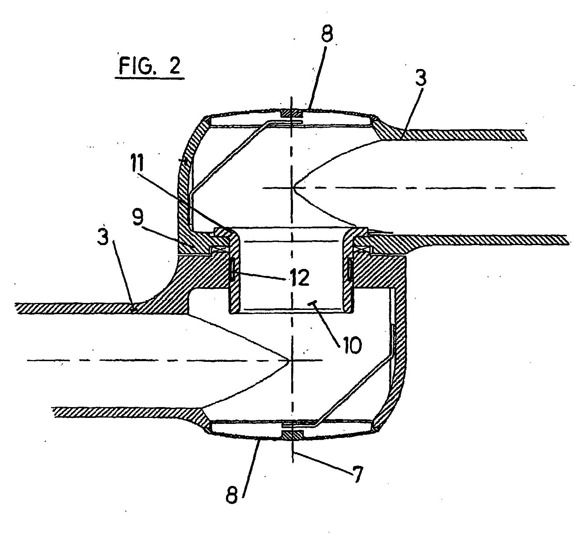

More specifically and as seen in figure 2, in each joint (4), an imaginary vertical tilting axis (7) is arranged, both the upper and lower portion of said joint being accessible by means of respective covers (8) which can be coupled by pressure, and the two halves of the joint being assembled with rotational movement around each other, according to said axis (7), through a bearing (9) or the like, collaborating in said joint there is a bushing (10) jointly fixed to one of the two halves of the joint, preferably to the upper one and by means of a perimetral flange (11), and which, on its area facing the lower half of the joint, has a perimetral groove in which an inflatable chamber (12) is housed, which, when empty, permits the free rotation between the arms (3) and (3'), whereas when inflated, acts as a brake immobilizing them.

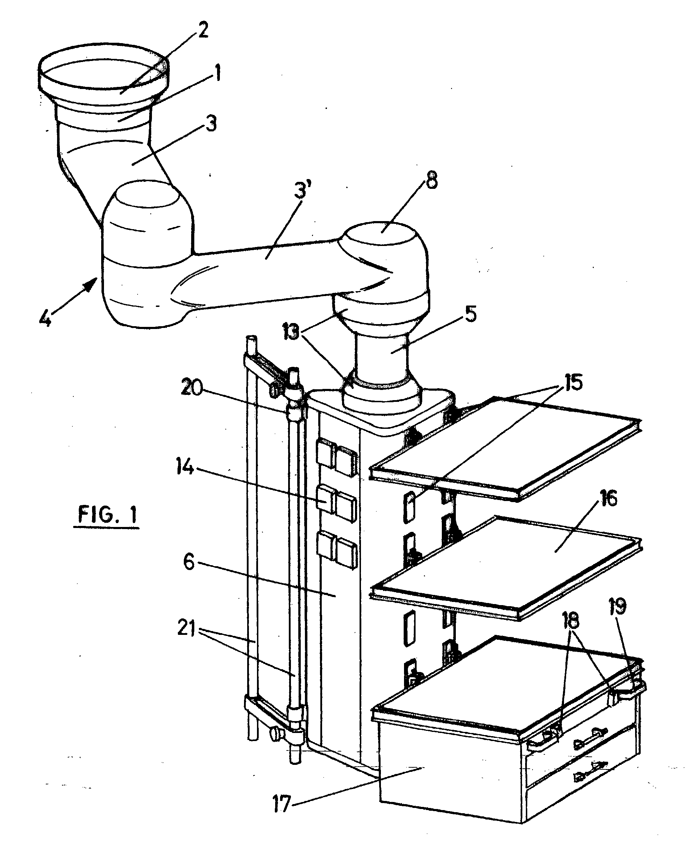

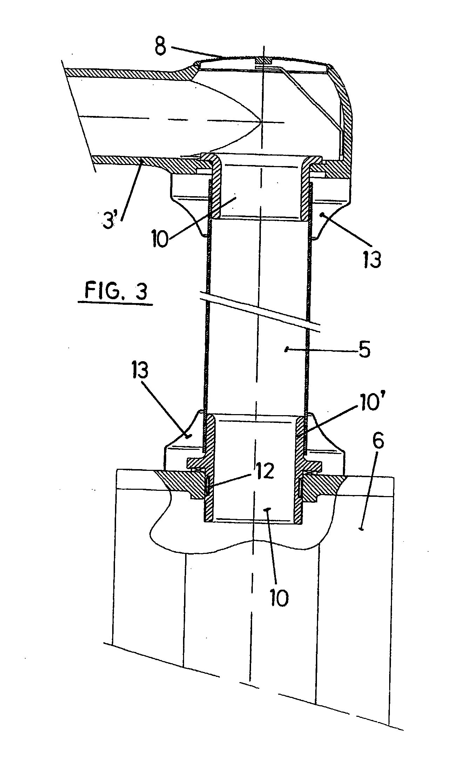

An identical bushing (10) connects the free end of the last arm (3') to the vertical telescopic connection (5), whereas the latter is connected to the container (6) through a similar although slightly modified bushing (10'), clearly seen in figure 3, bushings (10) and (10') inserted in the corresponding ends of the tube constituting the vertical connection (5), which in practice will be carried out in two telescopic tubes to obtain the intended vertical shifting of the container (6), said bushings (10) and (10') in any case being hidden by means of respective outer adornments (13), seen in both figure 1 and figure 2.

In each one of the lateral sides of the container (6), a plurality of small windows are arranged accompanied by respective collapsible covers (14), such that by eliminating any one of said covers (14), it is possible to arrange in the corresponding window fixing elements (15) susceptible to adopting any height setting and which permit fixing, extendible trays (16), chests of drawers (17) or any other type of accessory to the container (6), also at different height levels, by means of a simple coupling maneuver.

The control (18) for controlling the pneumatic element responsible for inflating emptying the inflatable chambers (12) for blocking the support joints will be arranged at the front of some of these elements, trays (16) or chest of drawers (17), and furthermore, the element occupying the lower end position, in the practical embodiment example in the figure, the chest of drawers (17) will incorporate handles (19) for pulling and orienting the container (6) with its accessories.

The last point to indicate is that, in correspondence with some of its edges, the container (6) can be provided with supports (20) for fixing extendible elements (21) which, as previously mentioned, in turn constitute support means for infusion pumps, drainage tubes, etc.

As inferred from the observation of the figures, there is an overwhelming predominance of rounded shapes and edges on the support, while at the same time a perfect superficial continuity is arranged in the coupling areas between the different mobile elements, which makes said support not only have an aesthetic attractiveness, with a perfect finish, but the maintenance thereof in optimal hygienic conditions is also very easy, with the particularity that when the vertical and telescopic connection (5) is in maximum retraction state, the container (6) with its accessories is located close to the ceiling of the room, precisely in the place where the support least obstructs when it is not being used.

## Claims

1. A medical supply support of the type intended for housing different types of instruments and to supply intakes of different fluids as well as for electric power, **characterized in that** it is constituted of a head (1) provided with means for fixing the head to the ceiling of the room where it is incorporated, a head to which a first horizontal arm (3) is hinged, and to this arm, another or more also horizontal and hinged arms (3), the last of which is finished in a vertical and telescopic connection (5) connecting said arms to a vertically elongated container (6) susceptible to being adapted in both this direction and in the transversal direction and with any orientation, with the particularity that both the head and the arms, the hinge joints (4) between them and the vertical and telescopic connection (5) are hollow, permitting passage through them of the channels corresponding to the different intakes for fluids, electricity or others, from the ceiling of the room.

2. A medical supply support according to claim 1, **characterized in that** each arm is finished on its ends with respective semi-joints for coupling to another arm, to the head or to the vertical telescopic connection, joints in which an imaginary vertical tilting axis (7) is arranged and which are accompanied by an inner bushing (10) fixed to one of the semi-joints, preferably to the upper one, and in its area facing the lower semi-joint, provided with a perimetral groove in which an annular and inflatable chamber (12) is housed, which acts as a brake or blocking element for the joint when it receives a suitable fluid pressure.

3. A medical supply support according to previous claims, **characterized in that** each joint incorporates a pair of end covers (8), an upper one and a lower one, coupled by pressure, for access to the inside thereof.

4. A medical supply support according to previous claims, **characterized in that** the windows of one of the sides of the container can be selectively used after removing the corresponding removable covers (14) for incorporating respective fixing elements (15) which permit coupling extendible trays (16) and/or chests of drawers (17) or other storage elements at different height levels selected by the user.

5. A medical supply support according to previous claims, **characterized in that** on the front edge of one of the trays or chests of drawers, preferably the one occupying the lower end position on the container, the control (18) is arranged for the inflating for the inflatable chambers for blocking the hinged joints, as well as handles (19) for pulling and orienting the entire container.

6. A medical supply support according to previous claims, **characterized in that** in correspondence with at least one of its edges, the container incorporates supports (20) for fixing extendible elements (21) which permit placing infusion pumps, drainage tubes or other accessories.
